# EUROPÄISCHE PATENTSCHRIFT

(11) **EP 0 854 152 B1**
(45) Veröffentlichungstag und Bekanntmachung des Hinweises auf die Patenterteilung: **12.03.2003**
(21) Anmeldenummer: 97122249.2
(22) Anmeldetag: 17.12.1997
(51) Int. Cl.: C07K 16/18, G01N 33/577, G01N 33/68, C07K 1/14

(54) **Antikörper und immunoassay zum Nachweis von MIA**
Antibodies and immunoassay for the detection of MIA
Anticorps et dosage immunologique pour la détection de MIA

(30) Priorität: 20.12.1996 DE 19653358
(43) Veröffentlichungstag der Anmeldung: 22.07.1998
(73) Patentinhaber: Roche Diagnostics GmbH, 68298 Mannheim (DE)
(72) Erfinder: Kaluza, Brigitte, Dr., 83670 Bad Heilbrunn (DE); Bartke, Ilse, Dr., 82347 Bernried (DE); Lenz, Helmut, Dr., 82327 Tutzing (DE); Kaufmann, Martin, 82362 Weilheim (DE); Büttner, Reinhard, Dr., 93090 Bach/Donau (DE); Bosserhoff, Anja-Katrin, Dr., 93059 Regensburg (DE)

(56) Entgegenhaltungen:
- WO-A-95/03328
- A. BLESCH ET AL.: "Expression and function of a novel, potent malignant melanoma cell growth inhibitor (MIA)." PROCEEDINGS OF THE AMERICAN ASSOCIATION FOR CANCER RESEARCH, Bd. 35, März 1994, VSA, Seite 567 XP002060475
- A. BOSSERHOFF ET AL.: "Melanoma-inhibiting activity, a novel serum marker for progression of malignant melanoma." CANCER RESEARCH, Bd. 57, Nr. 15, 1.August 1997, BALTIMORE, MD, VSA, Seiten 3149-3153, XP002060476
- P. HAU ET AL.: "Evidence for an alternative splice product of MIA/CD-RAP." PROCEEDINGS OF THE AMERICAN ASSOCIATION FOR CANCER RESEARCH, Bd. 38, März 1997, VSA, Seite 204 XP002060477

## Beschreibung

Gegenstand der vorliegenden Erfindung sind hochaffine, monoklonale Antikörper gegen MIA (Melanoma inhibitory activity), deren Verwendung zum Nachweis von MIA, sowie Immunoassays zum Nachweis von MIA.

Bei MIA (Melanoma Inhibitory Activity) handelt es sich um ein 11 kDa großes, lösliches Protein, das von malignen Melanomzellen sekretiert wird. Das maligne Melanom ist ein von den pigmentbildenden Zellen der Haut, den Melanocyten, ausgehender bösartiger Tumor, der stark zur Metastasenbildung neigt. Die Heilungschancen hängen vom Ausmaß der Metastasierung ab, wobei zur Beurteilung des Tumors gemäß den Empfehlungen der Deutschen Gesellschaft für Dermatologie eine Einteilung in die klinischen Stadien I bis IV erfolgt. Im Stadium I handelt es sich um einen Primärtumor ohne erkennbare Metastasen. Die Tumordicke beträgt bis zu 1,5 mm. Dieser Primärtumor kann operativ entfernt werden. Im Stadium II haben sich bereits in der unmittelbaren Umgebung des Tumors Metastasen gebildet (regionale Lymphknotenmetastasierung). Die Tumordicke im Stadium II beträgt 1,5 bis 4 mm. Die Heilungschancen im Stadium III sind aufgrund der bereits erfolgten Metastasenbildung und des Lymphknotenbefalls in weiter entferntem Gewebe nur noch in geringem Ausmaß gegeben. Im Stadium IV bestehen infolge der Fernmetastasierung und des vollständigen Befalls von Lymphknoten mit Metastasen praktisch keine Heilungschancen mehr.

Beim malignen Melanom handelt es sich um eine Krebsart, an der mit stark ansteigender Tendenz immer mehr Menschen erkranken. Nach der Entfernung des Primärmelanoms (Stadium I) ist es für den Patienten von besonderer Bedeutung, sicherzustellen, daß das Melanom vollständig entfernt wurde und daß keine Metastasen gebildet wurden. Die klassische Nachsorge besteht bisher darin - neben der regelmäßigen visuellen Begutachtung der Haut hinsichtlich äußerlich wahrnehmbarer Veränderungen " mit physikalischen Messverfahren wie Röntgen, Sonographie, Computertomographie und gelegentlich sogar Positronen-Emissionstomographie nach Metastasen im Körperinneren zu suchen, wobei bei von diesen Verfahren Tumoren unter 0,5 cm Durchmesser, auch bei multipler Metastasierung, noch nicht sicher erfaßt werden. In der Vergangenheit wurden bisher die Parameter S100 (Guo et al., European J. Cancer 1995, Vol 31 A, No.6, S. 924 - 928; v. Schoultz et al., Melanoma Research 1996, Vol 6, S. 133 - 137; Schultz et al., Zeitschrift f. Hautkrankheiten, H+G 1996, Vol 11 (71), S. 870 - 884) oder sICAM-1 (Soluble intercellular adhesion molecule 1) als Tumormarker verwendet. Der Nachweis von S100 oder sICAM-1 korreliert jedoch nicht zuverlässig mit dem Vorhandensein eines Melanoms. Nach Viac et al. (Cancer Lett. 1993, 72, S. 191-194), scheint insbesondere der Nachweis des Markers sICAM-1 kaum klinische Signifikanz für den Nachweis von Melanomen zu besitzen.

Ein diagnostisch eindeutiger Parameter, der zuverlässig die Bildung von Metastasen des malignen Melanoms vor allem nach Entfernung des Primärmelanoms ausschließt oder bestätigt, konnte bisher nicht gefunden werden. Auch ein zuverlässiger diagnostischer Test zum Nachweis des malignen Melanoms ist im Stand der Technik bisher nicht bekannt.

In der WO 95/03328 wird auf die wachstumshemmenden Eigenschaften von MIA insbesondere auf Tumorgewebe hingewiesen, die MIA vor allem als Therapeutikum interessant erscheinen lassen. In dieser Publikation wird prinzipiell auf die Machbarkeit von Antikörpern gegen dieses Protein verwiesen.

In Blesch et al. (Proceedings of the American Association for Cancer Research, Band 35, März 1994, Seite 567, Nr. 3379) wird die rekombinante Expression von MIA und die Detektion des exprimierten Proteins mittels eines MIA-Peptid spezifischen Antikörpers beschrieben. Es wird darauf hingewiesen, dass MIA möglicherweise für die therapeutische Behandlung des malignen Melanoms eingesetzt werden kann. Ein diagnostischer Nachweis von MIA in einer Patienten-Probe wird nicht beschrieben.

Aufgabe war es, einen zuverlässigen Parameter für den diagnostischen Nachweis des malignen Melanoms zu finden und einen verbesserten diagnostischen Test zu entwickeln, mit Hilfe dessen es möglich ist, den klinischen Verlauf der Erkrankung und den Heilungsprozess zuverlässig zu verfolgen und ein möglichst sensitives Screening zum Nachweis eines malignen Melanoms und dessen Metastasen durchzuführen.

Gelöst wird die Aufgabe durch monoklonale Antikörper gegen natives MIA, die mit hoher Affinität an dieses Protein binden.

Die monoklonalen Antikörper können in allen dem Fachmann geläufigen Tests zum Nachweis eines Proteins verwendet werden. Bei der bevorzugten Testführung mit zwei Antikörpern (Sandwich-Test) kann der Test einstufig, also ohne zusätzlichen Waschschritt zum Entfernen der ungebundenen Probenbestandteile durchgeführt werden. Diese vereinfachte Testführung ist insbesondere bei Screening-Tests, bei denen eine Vielzahl von Proben in möglichst rascher Folge getestet werden soll ein großer Vorteil.

Die Affinitätskonstanten der monoklonalen Antikörper für MIA können beispielsweise mit dem BIAcore®-System der Firma Pharmacia bestimmt werden. Zur Bestimmung der Affinität kann mit rekombinanten Methoden hergestelltes, humanes natives MIA verwendet werden. Die monoklonalen Antikörper besitzen erfindungsgemäß eine Affinität zu humanem nativen MIA von mindestens 10⁸ l/mol, besonders bevorzugt von mindestens 10⁹ l/mol. Diese hochaffinen monoklonalen Antikörper sind ausgezeichnet für Einschritteste geeignet, bei denen im allgemeinen eine relativ kurze Inkubation der Probe mit dem monoklonalen Antikörper erfolgt.

Die erfindungsgemäßen monoklonalen Antikörper können allen möglichen Immunglobulinklassen (Ig) angehören. Bevorzugt gehören die monoklonalen Antikörper der IgG1-Klasse an. Die Kopplung von weiteren Komponenten wie beispielsweise Markierungen wie Enzyme oder Haptene oder Bindepartner, die zur Bindung des Antikörpers an eine Festphase bei heterogenen Immunoassays benötigt werden, kann bevorzugt an IgG1-Antikörper erfolgen. Auch die Herstellung von Antikörper-Fragmenten wie beispielsweise F(ab')₂-, Fab'- oder Fab-Fragmenten ist bei der IgG1-Klasse unproblematisch.

Erfindungsgemäß werden unter der Bezeichnung "monoklonaler Antikörper" sowohl der vollständige Antikörper als auch alle bei Immuntests und sonstigen Verwendungen gängigen Fragmente davon, wie F(ab')₂-, Fab'-oder Fab-Fragmente, verstanden. Umfaßt sind auch solche Antikörper, die durch Veränderung der monoklonalen Antikörper hergestellt wurden, solange die Antigenbindeeigenschaft nicht entscheidend beeinflußt wurde. Beispielsweise können durch gentechnologische Maßnahmen Teile der normalerweise in Mäusen hergestellten monoklonalen Antikörper durch entsprechende humane Antikörpersequenzen ersetzt werden, um unspezifische Bindungen im Immunoassay zu minimieren. Verfahren zur Herstellung solcher chimärer monoklonaler Antikörper sind dem Fachmann beispielsweise aus Antibody Engineering, J. Mc Cafferty, H.R. Hoogenboom und D.J. Chiswell, The Practical Approach Series, Series Editor: B.D. Harnes, Oxford University Press, 1996, bekannt.

Die erfindungsgemäßen monoklonalen Antikörper können beispielsweise aus den Zellinien MAK<hMIA>M-2.F7.3B1 und MAK<hMIA>M-1.A12.9A1, hinterlegt am 10.12.96 mit den Hinterlegungsnummern DSM ACC 2292 und DSM ACC 2291 bei der DSMZ (Deutsche Sammlung von Mikroorganismen und Zellkulturen GmbH, Mascheroder Weg 1b, D-38124 Braunschweig), hergestellt werden.

Gegenstand der Erfindung sind weiterhin Antikörper, bevorzugt monoklonale Antikörper, die in äquivalenter Weise an MIA binden, wie die monoklonalen Antikörper <hMIA>M-2.F7.3B1 und <hMIA>M-1.A12.9A1. Unter "in äquivalenter Weise binden" wird verstanden, daß diese Antikörper mit gleich hoher oder vergleichbar hoher Affinität an MIA wie die hinterlegten monoklonalen Antikörper binden. Dies kann beispielsweise durch entsprechende Versuche am BIAcore® festgestellt werden.

Gegenstand der Erfindung sind weiterhin Antikörper, bevorzugt monoklonale Antikörper, die an die gleichen Epitope an MIA binden, wie die hinterlegten Antikörper <hMIA>M-2.F7.3B1 und <hMIA>M-1.A12.9A1. Dies kann durch Bestimmung der Kreuzreaktivität der Antikörper, beispielsweise mit dem BIAcore®-System festgestellt werden. Bevorzugt besitzen diese Antikörper ebenfalls eine hohe Affinität zu MIA.

Die erfindungsgemäßen monoklonalen Antikörper können in an sich bekannter Weise durch Immunisierung mit isoliertem MIA (aus humanem Gewebe isoliert oder rekombinant) in geeigneten Versuchstieren wie beispielsweise Mäuse, Ratten, Kaninchen und anschließender Fusion der Milzzellen der immunisierten Tiere mit Myelomzellen hergestellt werden. Neben Milzzellen als Lymphocyten-Quelle können auch periphere Blut-Lymphocyten (PBL) oder Lymphknoten-Zellen von immunisierten Tieren (bevorzugt: Maus oder Ratte) verwendet werden.

Alternativ können auch Lymphocyten von humanen Spendern (Gewebeproben von Melanom-Patienten), die Antikörper gegen MIA entwickelt haben, immortalisiert werden. Solche Anti-MIA-Antikörper produzierenden Lymphocyten können entweder durch Fusion mit einer humanen Myelom-Zellinie oder durch Epstein-Barr-Virus (EBV)-Transformation zu antikörper-produzierenden Hybridom-Zellen immortalisiert werden (Monoclonal Antibody and Immunosensor Technology, A.M. Campbell, Elsevier Verlag 1991; Monoklonale Antikörper, J.H. Peters, H. Baumgarten, Springer Verlag 1990; Monoclonal Antibody Production Techniques and Applications, ed. Lawrence B. Schook, Marcel Dekker Verlag 1987).

Werden Lymphocyten von humanen Spendern verwendet, die als Folge eines Melanoms Antikörper gegen MIA entwickelt haben, so ist die Verwendung von geeignetem MIA beim Screening-Verfahren nach Antikörper-sezemierenden Fusionszellen zu beachten. Als geeignetes MIA wird bevorzugt renaturiertes, also natives, nicht denaturiertes MIA eingesetzt.

Erfolgt die Herstellung der erfindungsgemäßen Antikörper nach dem zuerst genannten Verfahren, das heißt durch Immunisierung von Tieren und anschließendem Fusionieren der Lymphocyten mit Myelomzellen, so kommt der Herkunft bzw. der Aufreinigung des Immunogens und dem anschließenden Screening-Verfahren eine besondere Bedeutung zu.

So konnten durch Immunisierung von Mäusen mit Peptiden, die aus der N- und C-terminalen Aminosäuresequenz von MIA abgeleitet waren (AS-Position 1-12 bzw. 94-107), zwar monoklonale Antikörper gewonnen werden. Die so erzeugten Antikörper erkannten jedoch natives MIA nur mit einer Affinität im Bereich von 10⁶ l/mol (BIAcore®-System). Vermutlich erkannten diese Antikörper lineare Epitope und keine Konformationsepitope. Wurde die Immunisierung mit rekombinantem, zur nativen Konformation renaturiertem MIA durchgeführt (entsprechend dem in der WO 95/03328 beschriebenen Verfahren), so konnten monoklonale Antikörper hergestellt werden, deren Affinitätskonstante bei der Bindung von MIA im BIAcore®-System ebenfalls nur 10⁶ bis 10⁷l/mol betrug. Die Aufreinigung bzw. das Screening nach Hybridomlinien, die die gewünschten Antikörper gegen MIA sezernierten, erfolgte in diesem Fall mit Hilfe von MIA, welches über Spontanadsorption an Mikrotiter-Platten immobilisiert worden war.

Die erfindungsgemäßen hochaffinen monoklonalen Antikörper gegen natives MIA konnten dadurch erhalten werden, daß als Immunogen rekombinantes humanes natives MIA eingesetzt wurde. Das MIA wurde aus dem Expressionsplasmid p11379 hergestellt, das unter der Hinterlegungsnummer DSM 9267 am 29.06.1994 bei der Deutschen Sammlung von Mikroorganismen und Zellkulturen GmbH hinterlegt wurde (siehe auch WO 95/03328). MIA wurde unter renaturierenden Bedingungen aufgearbeitet, so daß natives, nicht denaturiertes MIA erhalten wurde. Die Aufreinigung bzw. das Screening nach Hybridomzellinien, die die gewünschten Antikörper gegen MIA sezernierten, erfolgte in diesem Fall mit Hilfe von biotinyliertem MIA, welches über Streptavidin an Mikrotiter-Platten gekoppelt worden war. Offensichtlich bleibt bei der Biotinylierung die native Konformation von MIA erhalten, so daß bei diesem Screening-Verfahren hochaffine Antikörper gegen humanes natives MIA erhalten werden konnten. Im Gegensatz dazu scheint das MIA-Protein bei der weiter oben genannten Screening-Methode mittels MIA, das über Spontanadsorption an KunststoffOberflächen gebunden wird, zu denaturieren, so daß Antikörper, die (native) Konforrnationsepitope auf dem MIA-Protein erkennen, nicht erfaßt werden.

Gegenstand der Erfindung ist daher auch ein Verfahren zur Herstellung von hochaffinen monoklonalen Antikörpern gegen MIA, das dadurch gekennzeichnet ist, daß die Immunisierung mit rekombinant hergestelltem, humanen, nativen MIA erfolgt und das Screening der erhaltenen Hybridomzellinien mit Hilfe von MIA durchgeführt wird, das in nativer Konformation vorliegt.

Mit diesem Verfahren können bevorzugt monoklonale Antikörper mit einer Affinitätskonstante bezüglich der Bindung von MIA von mindestens 10⁸ l/mol und besonders bevorzugt von mindestens 10⁹ l/mol hergestellt werden.

Ein weiterer Gegenstand der Erfindung ist die Verwendung der erfindungsgemäßen monoklonalen Antikörper zum diagnostischen Nachweis von MIA in Proben, bevorzugt humanen Proben wie beispielsweise Plasma, Serum, Blut, Speichel, Urin, Liquor, Lymphe, Muttermilch, Zysten. Seminalflüssigkeit, Gewebehomogenaten, Gewebeschnitten und Biopsiematerial.

Gegenstand der Erfindung ist weiterhin ein Verfahren zur Bestimmung von MIA durch Inkubation der Probe mit mindestens einem erfindungsgemäßen monoklonalen Antikörper. Alle gängigen, dem Fachmann geläufigen Methoden zum Nachweis eines Proteins, wie beispielsweise kompetitive Teste oder direkte Teste wie Sandwich-Teste, sind geeignet. Neben heterogenen Testen, bei denen die Assaykomponenten an eine Festphase gekoppelt sind und eine Trennung von fester und flüssiger Phase erfolgt, können auch homogenen Teste, sofern sie zum Nachweis eines Proteins geeignet sind, verwendet werden. Beispiele hierfür sind nephelometrische oder turbidimetrische Tests wie Latexagglutinationstests oder TINIA (turbidimetrische Inhibitionsimmunoassays). Neben den sogenannten Naßtesten, bei denen die Testreagenzien in flüssiger Phase vorliegen, können auch alle gängigen Trockentestformate, die zum Nachweis eines Proteins geeignet sind, verwendet werden. Bei diesen Trockentests oder Teststreifen sind die Testkomponenten auf einem Träger aufgebracht. Solche Trockentests sind beispielsweise in der EP-A-0 186 799 beschrieben.

Zu den bevorzugten Testführungen gehört ein Sandwich-Test, bei dem die Probe gleichzeitig mit zwei erfindungsgemäßen monoklonalen Antikörpern, von denen einer mit der Festphase bindefähig ist und der anderer eine Markierung trägt, inkubiert wird. Bei dieser Testführung ist es wichtig, daß die beiden eingesetzten monoklonalen Antikörper Epitope des MIA erkennen, die auf dem MIA-Protein räumlich so lokalisiert sind, daß beide Antikörper gleichzeitig an MIA binden können. Für diese Testführung sind daher bevorzugt verschiedene Epitope, das heißt Epitope, die unterschiedliche Strukturen besitzen, geeignet. Es können jedoch auch Antikörper eingesetzt werden, die gleiche Epitope erkennen, wenn diese Epitope so lokalisiert sind, daß beide Antikörper gleichzeitig an MIA binden können. Nach der Trennung der festen von der flüssigen Phase erfolgt die Bestimmung der Markierung nach dem Fachmann geläufigen Verfahren. Um quantifizierbare Testergebnisse zu erhalten, wird der Fachmann im allgemeinen gleichzeitig mit der Vermessung der Proben einige Standards mit bekannten MIA-Konzentrationen bestimmen und anhand der so erhaltenen Eichkurve bzw. -gerade die MIA-Konzentration in der Probe bestimmen.

Die erfindungsgemäßen Tests zum Nachweis von MIA ermöglichen einen sicheren Nachweis über das Vorliegen eines malignen Melanoms. In einer klinischen Studie konnte ein eindeutiger Zusammenhang zwischen erhöhter MIA-Konzentration im Serum und fortgeschrittenem Erkrankungsstadium festgestellt werden (siehe Beispiel 6). Ausgehend von einem Cut-Off-Wert von 6,5 ng MIA pro ml, (95 % eines gesunden Normalkollektivs liegen unter diesem Wert), zeigten bereits 14 % der Patienten im Stadium I erhöhte Serumkonzentrationen an MIA. Im Stadium II wiesen 40 % der Seren erhöhte MIA-Konzentrationen auf, im Stadium III und IV sogar 100 % der Patientenseren.

Die nachfolgenden Beispiele erläutern die Erfindung weiter.

### Beispiel 1:

### Herstellung des Immunogens

Das Immunogen, das heißt, rekombinantes MIA wurde aus Einschlußkörperchen ("inclusion bodies") gewonnen, in denen das MIA unlöslich vorlag. Diese wurden dadurch hergestellt, daß das Expressionsplasmid p11379 in einen geigneten Expressionsstamm des Bakteriums *Escherichia coli* überführt und dem Stand der Technik entsprechend fermentiert und durch spezifische Induktion die Produktion des MIA induziert wird und anschließend die Einschlußkörperchen durch Aufbrechen der Zellen und Zentrifugation gewonnen werden. Dieses Verfahren ist dem Fachmann bekannt und braucht daher hier nicht näher erläutert zu werden. Da das MIA nur als unlösliches Aggregat vorlag, mußte es in eine lösliche, native Form überführt werden. Dies gelang erfindungsgemäß erst nach der Einführung von zusätzlichen, stabilisierenden Substanzen.

Dazu wurden 1,5 g der Einschlußkörperchen in 150 ml eines Puffer, enthaltend 6 mol/l Guanidiniumhydrochlorid, 100 mmol/l Tris/HCl, pH 8,0, 200 mmol/l Dithioerythrol (DTE) und 1mmol/l Ethylendiamintetraessigsäure (EDTA) durch Ultrabeschallung gelöst und eine Stunde bei 25 °C inkubiert. Anschließend wurde der pH-Wert durch Zugabe von 25 %iger (v/v) HCl auf einen Wert von 3,0 abgesenkt. Danach wurde die Lösung über Nacht gegen den obigen Puffer, jedoch eingestellt auf einen pH-Wert von 3,0 sowie ohne Zusatz von DTE, dialysiert. Das so gewonnene Dialysat wurde im Verhältnis 1:200 in einen Puffer, enthaltend 1 mol/l Arginin, 1 mmol/l EDTA, 5 mmol/l reduziertes Glutathion (GSH) und 500 µmol/l oxidiertes Glutathion (GSSG), verdünnt und 24 Stunden bei 25 °C inkubiert.

Für die Aufreinigung wurde diese Lösung durch Zugabe von festem Ammoniumsulfat auf eine Ammoniumsulfatkonzentration von 1,4 mol/l gebracht und mit einer in 1,4 molarem Ammoniumsulfat equilibrierten Austauschermatrix (Fractogel TSK-Butyl-650 [M][Merck KGaA, 16873]) versetzt und eine Stunde inkubiert. Der Austauscher wurde abfiltriert, mit 1,4 molarem Ammoniumsulfat in 20 mmol/l Tris pH 7,0 gewaschen und in eine Glassäule gefüllt. Die Elution des MIA-Proteins erfolgte durch einen fallenden Gradienten von Ammoniumsulfat von 1,4 mol/l in 20mmol/l Tris pH 7,0 auf 20mmol/l Tris pH 7,0.

Das so gewonnene MIA wurde als Immunogen eingesetzt.

### Beispiel 2:

### Herstellung der monoklonalen Antikörper

BALB/C Mäuse wurden mit 100 µg rec. nativem MIA intraperitoneal immunisiert. Die Erstimmunisierung erfolgte in komplettem Freund'schen Adjuvants (CFA) -alle weiteren Immunisierungen wurden in inkomplettem Freund schen Adjuvants (IFA) durchgeführt. Die Immunisierung erfolgte in ca. 4-wöchigem Abstand. Die letzten drei Immunisierungen wurden im Abstand von je einem Tag intravenös (i.v.) durchgeführt. Nach der letzten i.v. Immunisierung erfolgte die Immortalisierung der Milzzellen der immunisierten Tiere mit der Myelomzellinie P3X63.Ag8.653.

Die Fusion der Milzzellen mit der Myelomizellinie wird nach dem Standardverfahren gemäß Goding, J.W., J.of Imm. Meth., 39 (1980) 285-308 durchgeführt. Das Fusionsverhältnis Milzzellen:Myelomzellen ist dabei 1:1. Die Fusionsprodukte werden auf 24er Kulturschalen (Nunc) mit HFCS (Boehringer Mannheim GmbH, Kat.-Nr.1363735) ausgesät. Positive Primärkulturen werden zwei Wochen nach der Fusion mit Hilfe eines fluoreszenzaktivierten Zellsorters (Beckton Dickinson) kloniert. Die Zellen werden dabei einzeln in 96er Mikrotiterplatten abgelegt und mit Nutridoma CS- Medium (Boehringer Mannheim GmbH Kat.-Nr. 1363743) gefüttert. Als Kulturmedium wird anschließend handelsübliches RPMI 1640 mit 10%igem fötalem Kälberserum verwendet.

### Beispiel 3:

### Bestimmung der Spezifität der produzierten Antikörper

Um die Spezifität der Antikörper im Kulturüberstand der Hybridomzellen zu erfassen, wird ein Enzyme-linked-Immuno-Sorbent-Assay (ELISA) verwendet.

Dazu werden 96er Streptavidin-beschichtete Mikrotiterplatten-Platten (Boehringer Mannheim Katalog Nr.1734776) mit 50µl rekombinantem nativen biotinylierten MIA (1 µg/ml) in Carbonatpuffer (Boehringer Mannheim GmbH, Kat.-Nr. 726559) 1h bei 37 C beschichtet, mit 50µl Kulturüberstand 2h bei 37 C inkubiert und mit 3x 250 µl PBS/0,05% Tween 20 gewaschen. Danach wird mit POD markiertem Schaf-Anti-Maus-IgG (Boehringer Mannheim) 60 Minuten bei 37 C inkubiert, mit 3x 250 µl PBS/0,05% Tween 20 gewaschen und die Nachweisreaktion mit 50 µl ABTS ( 1 mg/ml,Boehringer Mannheim, Katalog Nr. 1204 530) ausgelöst. Nach 20 Minuten Inkubation bei Raumtemperatur werden die Extinktionen in einem Photometer bei 405 nm bestimmt.

### Beispiel 4:

### Bestimmung der Affinitätskonstanten sowie der Geschwindigkeitskonstanten der Assoziation und Dissoziation der produzierten Antikörper

Die Bestimmung der Affinitätkonstanten sowie der Geschwindigkeitskonstanten der Assoziation und Dissoziation der produzierten Antikörper erfolgte mit BIAcore® der Firma Pharmacia Biosensor (BIA steht für Biospezifische Interaktions-Analyse). Das Messprinzip beruht auf der "Surface Plasmon Resonance". Die Messung wird auf einem Biosensor, dem sog. Sensorchip durchgeführt. Hierbei wird auf die mit carboxymethyliertem Dextran beschichtete Oberfläche eines Sensorchips (CM5, BIAcore® AB) ein polyclonaler Kaninchen-Antikörper gegen den Fcγ-Teil von Maus-IgG über seine Aminogruppen kovalent gekoppelt. Über diesen Sensorchip wird eine Lösung des zu bestimmenden Antikörpers geleitet, wobei der Antikörper durch nichtkovalente Wechselwirkungskräfte an den immobilisierten Fangantikörper gebunden wird. Im folgenden wird das zu untersuchende Antigen über den Sensorchip geleitet, welches dann durch ebenfalls nichtkovalente Wechselwirkungskräfte an den über den Fangantikörper immobilisierten Antikörper gebunden wird.

Durch die Bindung der einzelnen Komponenten erhöht sich die Massendichte auf der Oberfläche des Sensorchips, die vom Gerät in ein proportionales Messignal umgewandelt wird. Aus der zeitlichen Änderung des Signals, dem Sensorgramm, lassen sich die Geschwindigkeitskonstanten der Assoziation und Dissoziation und daraus die Affinitätskonstante berechnen.

Die Antikörper-Antigen-Komplexe lassen sich ohne Beeinträchtigung der an die Oberfläche gebundenen Fangantikörper mit einfachen Mitteln wieder ablösen, so daß an demselben Sensorchip weitere Bindungsexperimente unter identischen Randbedingungen durchgeführt werden können.

Zur Kopplung des Fangantikörpers an den Sensorchip (CM5, BIAcore® AB) wird eine Lösung des Antikörpers (BIA certified Rabbit anti Mouse Fcγ, BIAcore® AB) mit einer Konzentration von 60 µg/ml in 10 mM Natriumacetat-Puffer pH 5.0 mit einer Flußrate von 5 µl/min über den zuvor mit NHS/EDC aktivierten Sensorchip geleitet.

Danach werden die Antikörper zugegeben, so daß eine Zunahme der an die Oberfläche gebundenen Masse von mindestens 600 Resonanzeinheiten erfolgt. Die Bindung der Antigene an die Antikörper wird bei einer Flußrate von 10 µl/min verfolgt und die Geschwindigkeitskonstanten der Assoziation und der Dissoziation für die Bindung an die Antikörper aus den Setisorgrammen mit Hilfe einer Software des Herstellers (BIAevaluation 2.1, Pharmacia Biosensor) berechnet. Die Affinitätskonstante berechnet sich nach Ka = kon/koff. Die so ermittelten Werte der erfindungsgemäßen Antikörper mit MIA sind in der unten angegebenen Tabelle zusammengefaßt.

**Tabelle 1**

| Affinitätskonstanten der MIA-MAKs | | | |
|---|---|---|---|
| | MIA | | |
| Klon | kon | koff | Ka |
| | l/mol*s | 1/s | l/mol |
| 1.A12.9A1 | 9,3*10⁺⁵ | 4,2*10⁻⁴ | 2,2*10⁺⁹ |
| 2.F7.3B1 | 5,7*10⁺⁵ | 1,8*10⁻³ | 3,2*10⁺⁸ |

### Beispiel 5:

### ELISA-Test zum Nachweis von MIA

### 5.1. Herstellung der Einsatzstoffe für den ELISA

### a) MAK<MIA>M-2F7-IgG-Biotin(DDS)1:4

Das Verfahren wurde gemäß der in der Offenlegungsschrift DE 43 02 241 A1 beschriebenen Methode durchgeführt.
Monoklonales Anti-MIA-M-2F7-IgG (Boehringer Mannheim GmbH) wird in 0.1 M Kalium-Phosphat-Puffer, pH 8.4, zu einer Konzentration von 10 mg/ml gelöst. Man setzt die 4-fache molare Menge an Biotin-DDS (gelöst in Dimethylsulfoxid ad c= 9.5mg/ml ) hinzu. Nach 90 min Rühren bei 25°C wird der Ansatz durch Zugabe von 10 µl 1 M Lysin / ml Ansatz gestoppt. Man dialysiert gegen 25 mM Kaliumphosphat 50 mM NaCl, pH 7.0 und lyophilisiert das Produkt.

### b) MAK<MIA>M-1A12-IgG-POD(p)

Das Verfahren wurde wie in der EP 0 378 175 B1 beschrieben durchgeführt.
Die IgG-Fraktion des monoklonalen Anti-MIA-Antikörpers wurde aus Ascites-Flüssigkeit durch Ammoniumsulfat-Fällung und Chromatographie an DEAE-Ionentauscher nach A. Johnstone und R. Thorpe, Immunochemistry in Practice, Blackwell Scientific, 1987, Seiten 50 - 54, gereinigt. 50 mg gereinigtes IgG wurden mit S-Acetyl-thio-bemsteinsäureanhydrid umgesetzt und mit 50 mg vorpolymerisierter Maleimido-hexanoyl-Peroxidase gekoppelt, nach der in DE-A-38 25 735 beschriebenen Methode. Durch AcA 22-Chromatographie wurde ein Konjugatpool mit einem Molekulargewicht von 2-3- Millionen erhalten. Ausbeute ca. 30 mg Protein mit 13 KU Peroxidase(POD)-Aktivität.

### c) MIA-Protein, nativ

Die Herstellung des als Standardmaterials verwendeten reinen, nativen MIA Proteins wird im Beispiel 1 ausführlich beschrieben.

### d) Inkubationspuffer des MIA-ELISAs ( Gewichtsangaben / Liter )

| | |
|---|---|
| NaH₂PO₄ | 0.97 g |
| Na₂HPO₄ | 5.88 g |
| Di-Na-Tartrat | 46 g |
| Synperonic F 68 | 5 g |
| Rinder-IgG | 1 g |
| Rinder-Albumin | 2 g |

Der pH-Wert wird mit NaOH ad 7.4 eingestellt.

### 5.2. Beschreibung des ELISA

### Schritt I:

In die Vertiefungen einer mit Streptavidin beschichteten Mikrotiterplatte (Boehringer Mannheim GmbH, Id.Nr. 1 664 778) werden je 10 µl Standardlösung (rec.h-MIA in 50 mmol/l Hepes, 150 mmol/l NaCl, pH 7.0, Verdünnungsreihe mit 0, 3.13, 6.25, 12.5, 25, 50 ng/ml) oder 10 µl der zu bestimmenden Probe ( Serum, Plasma etc.) vorgelegt. In jede mit Standard oder Probe beschickte Vertiefung werden anschließend 200 µl einer Lösung von 1 µg/ml MAK<MIA>M-2F7-Bi und 25 mU/ml MAK<MIA>M-1A10-POD in Inkubationspuffer pipettiert. Die Mikrotiterplatte wird dann 45 Minuten bei Raumtemperatur (20 - 25°C) auf einem handelsüblichen Schüttelgerät für Mikrotiterplatten (z.B. IKA MTS 4) bei 500 rpm geschüttelt.

### Waschschritt :

Die Vertiefungen der Mikrotiterplatte werden sorgfältig entleert und dreimal mit je 300 µl Waschlösung (250 mg NaCl/l, 1 mg CuSO₄/l, Boehringer Mannheim GmbH. Id.Nr.1 059 475) gewaschen.

### Schritt II:

In jede der in Schritt 1 bearbeiteten Vertiefungen der Mikrotiterplatte werden 200 µl ABTS®-Substratlösung (2,2'-Azino-di[3-ethylbenzthiazolin-sulfonat] 1.9 mmol/l ABTS®, 100 mmol/l Phosphat-Citrat-Puffer, pH 4.4, Natriumperborat 3.2 mmol/l) pipettiert und 30 Minuten bei Raumtemperatur ( 20 - 25°C) auf dem Plattenschüttler bei 500 rpm inkubiert.

Die Extinktionswerte der sich ergebenden Farblösungen in den Vertiefungen werden mit einem für Mikrotiterplatten geeigneten Photometer bei 405 nm gemessen. Aus den Signalen und den Konzentrationswerten der Standardreihe wird eine Standardkurve erstellt, an der die Probenkonzentrationen abgelesen werden.

### Beispiel 6:

### Vergleich der Sensitivität zwischen MIA-, S100- und sICAM-1-Tests

Die Bestimmung der MIA-Konzentration erfolgte nach der in Beispiel 5 beschriebenen Vorgehensweise. Die S100-Konzentrationen wurden mit einem kommerziell erhältlichen immunoradiometrischen Test der Firma Byk-Sangtec Diagnostics, Dietzenbach, Deutschland (siehe auch Kato et al, Biomed Res. 1987, Vol 8, S. 119-125), nach Herstellerangaben durchgeführt. Die sICAM-1-Werte wurden mit dem Einschritt-ELISA-Kit der Firma Amersham, Braunschweig, Deutschland, gemäß den Vorschriften der Packungsbeilage ermittelt.

**Tabelle 2a**

| Ermittelte Wertebereiche: | | | |
|---|---|---|---|
| getestete Seren Kollektiv | MIA ng/ml | S100 ng/ml | sICAM-1 ng/ml |
| Normale | 1.8-7.6 ( n=72 ) | < 0.15 ( n= 13 ) | 220 - 466 ( n=9 ) |
| Sepsis | 1.4-6.8 ( n=50 ) | 0 - 0.3 ( n=10 ) | 161 - 927 ( n=7 ) |
| Melanom Stad. I | 2.9-7.7 ( n=14 ) | < 0.15 ( n=14 ) | -- |
| Melanom Stad. II | 3.6-8.1 ( n=5 ) | < 0.15 ( n=5 ) | -- |
| Melanom Stad. III | 7.5-24.7 ( n= 5 ) | 0 - 13.3 ( n=5 ) | 194 - 1430 ( n=14) |
| Melanom Stad. IV | 7.5-64.8 ( n=21 ) | 0 - 11.7 ( n=21) | 194 - 1430 ( n=14) |
| "n" bedeutet die Anzahl der getesteten Seren | | | |

Diese Ergebnisse zeigen, daß MIA ein ausgezeichneter Marker für die Beurteilung der fortgeschrittenen Erkrankung an malignem Melanom ist. MIA ist ein Marker mit ausreichender Sensitivität, um eine Einordnung der Tumorerkrankung in die Stadien I bis IV vorzunehmen. Diese Einteilung ist anhand der Marker S100 und sICAM-1 nicht möglich. MIA ist ein wesentlich zuverlässigerer Tumormarker als S100 und sICAM-1. Die Bestimmung von MIA wird durch Sepsis-Seren (im Gegensatz zu sICAM-1) nicht gestört.

**Tabelle 2b**

| Prozentsatz an getesteten Seren, die anhand der Testergebnisse aus Tabelle 2a in die Stadien I bis IV einzuordnen sind. | | | |
|---|---|---|---|
| Kollektiv | MIA % > cut-off (6.5 ng) | S100 %> cut-off (0.15 ng) | sICAM-1 %> cut-off ( 280 ng) |
| Normale | 2.7 | 0 | 33 |
| Sepsis | 0 | 20 | 71 |
| Melanom Stad. I | 14 | 0 | -- |
| Melanom Stad. II | 40 | 0 | -- |
| Melanom Stad. III | 100 | 60 | 43 |
| Melanom Stad. IV | 100 | 57 | 43 |

Die Daten zeigen, daß die MIA-Bestimmung zuverlässiger in den Stadien II - IV auf das mögliche Vorhandensein von Metastasen hinweist und so zu einer Absicherung weiterer diagnostischer Schritte und der Prognose herangezogen werden kann. Ferner kann aus den Daten abgeleitet werden, daß MIA als Melanomspezifisches Protein wahrscheinlich besser in den Stadien III und IV mit der Tumormasse korreliert als S 100 oder sICAM-1.

## Patentansprüche

1. Monoklonale Antikörper gegen MIA, **dadurch gekennzeichnet, daß** sie eine Affinität zu MIA von mindestens 10⁸ l/mol besitzen.

2. Monoklonale Antikörper nach Anspruch 1, erhältlich durch Immunisieren eines geeigneten Versuchstieres mit rekombinant hergestelltem, humanen, nativen MIA und anschließendem Screening der erhaltenen Hybridomzellinien mit Hilfe von MIA, das in nativer Konformation vorliegt.

3. Monoklonale Antikörper gegen MIA mit einer Affinität zu MIA von mindestens 10⁸ l/mol, die von den Zellinien DSM ACC 2292 und DSM ACC 2291 produziert werden.

4. Monoklonale Antikörper gegen MIA mit einer Affinität zu MIA von mindestens 10⁸ l/mol, die in äquivalenter Weise an MIA binden wie die monoklonalen Antikörper, die von den Zellinien DSM ACC 2292 und DSM ACC 2291 produziert werden.

5. Monoklonale Antikörper gegen MIA mit einer Affinität zu MIA von mindestens 10⁸ l/mol, die an die gleichen Epitope binden wie die monoklonalen Antikörper, die von den Zellinien DSM ACC 2292 und DSM ACC 2291 produziert werden.

6. Verwendung eines monoklonalen Antikörpers gemäß einem der Ansprüche 1 bis 5 in einem diagnostischen Test zum Nachweis von MIA in einer Probe.

7. Verfahren zur Bestimmung von MIA durch Inkubation der Probe mit mindestens einem monoklonalen Antikörper nach einem der Ansprüche 1 bis 5.

8. Verfahren zur Bestimmung von MIA nach Anspruch 6 nach einem Sandwich-Test, **dadurch gekennzeichnet, daß** die monoklonalen Antikörper der Zellinien DSM ACC 2292 und DSM ACC 2291 verwendet werden.

9. Verfahren nach Anspruch 8, **dadurch gekennzeichnet, daß** monoklonale Antikörper verwendet werden, die in äquivalenter Weise an MIA binden oder die gleichen Epitope erkennen wie die monoklonalen Antikörper der Zellinien DSM ACC 2292 und DSM ACC 2291.

## Claims

1. Monoclonal antibodies against MIA wherein they have an affinity for MIA of at least 10⁸ l/mol.

2. Monoclonal antibodies as claimed in claim 1, obtainable by immunization of a suitable test animal with human, native MIA produced by recombinant means, and subsequent screening of the resulting hybridoma cell lines using MIA present in a native conformation.

3. Monoclonal antibodies against MIA with an affinity for MIA of at least 10⁸ l/mol, produced from the cell lines DSM ACC 2292 and DSM ACC 2291.

4. Monoclonal antibodies against MIA with an affinity for MIA of at least 10⁸ l/mol, binding in an equivalent way to MIA as the monoclonal antibodies produced from the cell lines DSM ACC 2292 and DSM ACC 2291.

5. Monoclonal antibodies against MIA with an affinity for MIA of at least 10⁸ l/mol, binding to the same epitopes as the monoclonal antibodies produced from the cell lines DSM ACC 2292 and DSM ACC 2291.

6. Use of a monoclonal antibody as claimed in one of the claims 1 to 5 in a diagnostic test for the detection of MIA in a sample.

7. Method of determination of MIA by incubation of the sample with at least one monoclonal antibody as claimed in one of the claims 1 to 5.

8. Method of determination of MIA as claimed in claim 6 according to a sandwich test wherein the monoclonal antibodies of the cell lines DSM ACC 2292 and DSM ACC 2291 are used.

9. Method according to claim 8, wherein monoclonal antibodies are used binding in an equivalent way to MIA or recognizing the sampe epitopes as the monoclonal antibodies of the cell lines DSM ACC 2292 and DSM ACC 2291.

## Revendications

1. Anticorps monoclonaux contre le MIA, **caractérisés en ce qu'**ils possèdent une affinité d'au moins 10⁸ l/moles avec le MIA.

2. Anticorps monoclonaux selon la revendication 1, pouvant être obtenus par immunisation d'un cobaye approprié à l'aide de MIA natif, humain, produit par recombinaison et suivie d'un triage des lignées cellulaires d'hybridomes de MIA, qui se présente sous une conformation native.

3. Anticorps monoclonaux contre le MIA présentant une affinité d'au moins 10⁸ l/moles avec le MIA, produits par les lignées cellulaires DSM ACC 2292 et DSM ACC 2291.

4. Anticorps monoclonaux contre le MIA présentant une affinité d'au moins 10⁸ l/moles avec le MIA, qui se fixent au MIA de manière équivalente aux anticorps monoclonaux produits par les lignées cellulaires DSM ACC 2292 et DSM ACC 2291.

5. Anticorps monoclonaux contre le MIA présentant une affinité d'au moins 10⁸ l/moles avec le MIA, qui se fixent aux mêmes épitopes que les anticorps monoclonaux produits par les lignées cellulaires DSM ACC 2292 et DSM ACC 2291.

6. Utilisation d'un anticorps monoclonal selon l'une des revendications 1 à 5 dans un test de diagnostic destiné à mettre en évidence la présence de MIA dans un échantillon.

7. Procédé pour déterminer la présence de MIA par incubation de l'échantillon avec au moins un anticorps monoclonal selon l'une des revendications 1 à 5.

8. Procédé pour déterminer la présence de MIA selon la revendication 6 d'après un test-sandwich, **caractérisé par** l'utilisation des anticorps monoclonaux des lignées cellulaires DSM ACC 2292 et DSM ACC 2291.

9. Procédé selon la revendication 8, **caractérisé par** l'utilisation d'anticorps monoclonaux qui se fixent au MIA de manière équivalente aux anticorps monoclonaux des lignées cellulaires DSM ACC 2292 et DSM ACC 2291 ou qui reconnaissent les mêmes épitopes que ceux-ci.
